# EUROPÄISCHE PATENTSCHRIFT

(11) **EP 1 373 241 B1**
(45) Veröffentlichungstag und Bekanntmachung des Hinweises auf die Patenterteilung: **28.12.2005**
(21) Anmeldenummer: 02719830.8
(22) Anmeldetag: 14.02.2002
(51) Int. Cl.: C07D 309/30, C07D 309/32

(54) **VERFAHREN ZUR HERSTELLUNG OPTISCH AKTIVER DIHYDROPYRONE**
METHOD FOR PRODUCING OPTICALLY ACTIVE DIHYDROPYRONES
PROCEDE DE PRODUCTION DE DIHYDROPYRONES OPTIQUEMENT ACTIVES

(30) Priorität: 22.02.2001 DE 10108470
(43) Veröffentlichungstag der Anmeldung: 02.01.2004
(73) Patentinhaber: Boehringer Ingelheim Pharma GmbH & Co.KG, 55216 Ingelheim am Rhein (DE)
(72) Erfinder: JÄGER, Burkhard, 55411 Bingen 13 (DE); SAUTER, Markus, 55457 Gensingen (DE)
(86) Internationale Anmeldenummer: PCT/EP2002/001534
(87) Internationale Veröffentlichungsnummer: WO 2002/068404

(56) Entgegenhaltungen:
- WO-A-95/14012
- WO-A-95/30670
- WO-A-99/12919
- GB-A- 1 355 667
- US-A- 4 661 628
- US-A- 6 147 095
- THAISRIVONGS S ET AL: "JOURNAL OF MEDICINAL CHEMISTRY, AMERICAN CHEMICAL SOCIETY. WASHINGTON, US" JOURNAL OF MEDICINAL CHEMISTRY, AMERICAN CHEMICAL SOCIETY. WASHINGTON, US, Bd. 39, Nr. 23, 1996, Seiten 4630-4642, XP002088571 ISSN: 0022-2623

## Beschreibung

Die Erfindung betrifft ein neues Verfahren zur Herstellung optisch aktiver Dihydropyrone, neue, über diese Synthese erhältliche Zwischenprodukte, sowie deren Verwendung als Ausgangsverbindungen bei der Herstellung pharmazeutisch wirksamer Verbindungen.

5,6-Dihydro-4-hydroxy-2-pyrone stellen wichtige Strukturelemente bei einer Vielzahl pharmazeutisch wirksamer Verbindungen dar. Von besonderem Interesse ist die Klasse der 5,6-Dihydro-4-hydroxy-2-pyron-sulfonamide, die als nicht-peptidische HIV-Protease-Inhibitoren zum Einsatz gelangen können. Ein besonders wirksames Beispiel für einen potenten und oral bioverfügbaren HIV-Protease-Inhibitor dieser Substanzklasse ist die Verbindung Tipranavir (PNU-140690), die die folgende Struktur aufweist

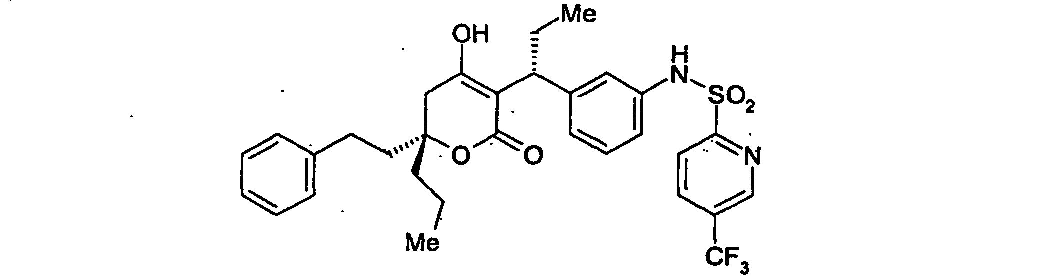

Diese wie auch andere strukturell ähnliche Verbindungen sind aus dem Stand der Technik bekannt (siehe beispielsweise *J. Med. Chem.* **1998**, *41,* 3467-3476).

Ein Schlüsselschritt bei der Synthese der vorstehend genannten, wie auch strukturell ähnlicher Verbindungen, ist die Umsetzung von 5,6-Dihydro-4-hydroxy-2-pyronen **1** mit geeignet substituierten Carbonylverbindungen **2** zu den Kondensationsprodukten **3**, wie er in Schema 1 skizziert ist.

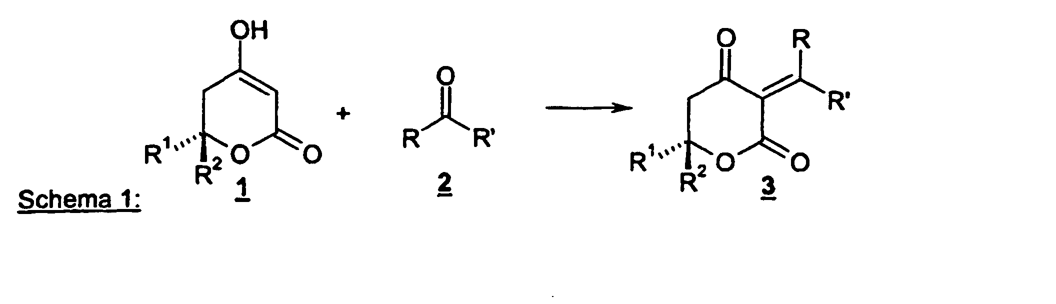

Die Bedeutung der voneinander verschiedenen Reste R¹ und R² ist der nachfolgenden detaillierten Beschreibung der vorliegenden Erfindung zu entnehmen. Die Bedeutung der Reste R und R' ist variabel und bestimmt sich durch das Substitutionsmuster der jeweiligen Zielverbindungen, wie sie dem Stand der Technik zu entnehmen sind.

Erfindungsgemäß bedeutsam ist, daß die in den Ausgangverbindungen **1** enthaltene chirale Information bei der in Schema 1 skizzierten Folgeumsetzung erhalten bleibt, wodurch den Verbindungen **1** eine zentrale Bedeutung bei der Synthese der vorstehend genannten pharmazeutisch wirksamen Verbindungen zukommt.

Es ist daher Aufgabe der vorliegenden Erfindung, ein Verfahren bereitzustellen, welches die Synthese von **1** **i**n hohen Ausbeuten, hoher Enantiomerenreinheit, unter geringstmöglichem technischen Aufwand und hoher Raum-Zeit-Ausbeute erfaubt.

Überraschenderweise wurde gefunden, daß vorstehend genannte Aufgaben der vorliegenden Erfindung gelöst werden können, wenn die chirale 5,6-Dihydro-4-hydroxy-2-pyrone **1** gemäß der in Schema 2 skizzierten Vorgehensweise bereitgestellt werden.

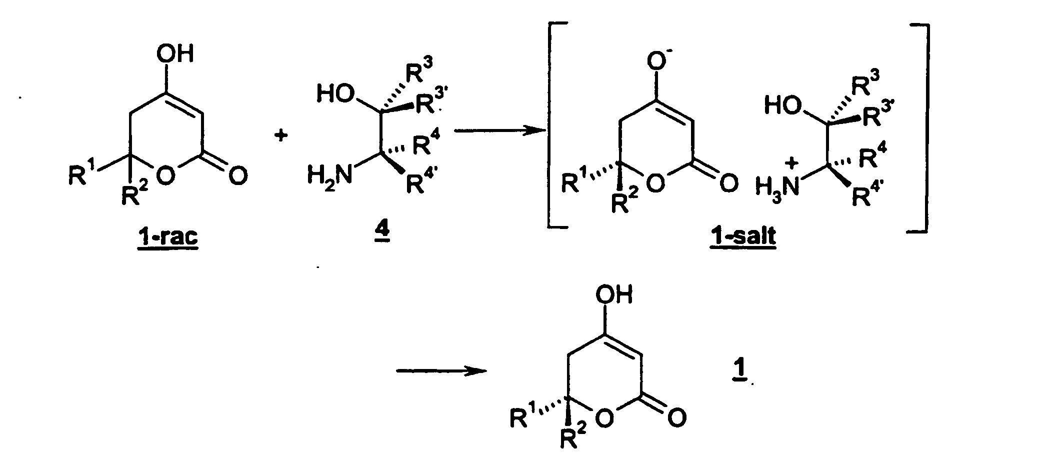

### Schema 2:

Racemisches 5,6-Dihydro-4-hydroxy-2-pyrone **1-rac** wird dazu mit chiralen Aminoalkoholen **4** in die Salze **1-salt** überführt. Diese lassen sich überraschenderweise in Form kristallisierender Verbindungen erhalten und so in guten Ausbeuten und hoher Reinheit isolieren. Je nach Wahl der optisch aktiven Aminoalkohole **4** kristallisieren die Salze **1-salt** der R- oder S-konfigurierten Verbindungen **1**. Die Salze **1-salt'** der nicht gewünschten Enantiomere von **1** bleiben bei diesem Reaktionsschritt in Lösung. Aus den kristallisierten Salzen **1-salt** werden die optisch aktiven Zielverbindungen freigesetzt.

Im Rahmen der vorliegenden Erfindung ist eine Bezugnahme auf Verbindungen der Formel **1** stets als Bezugnahme auf die optisch aktiven Verbindungen **1** zu verstehen, wohingegen eine Bezugnahme auf Verbindungen der Formel **1-rac** als Bezugnahme auf das racemische Gemisch der Verbindungen der Formel **1** zu verstehen ist.

Dementsprechend betrifft die vorliegende Erfindung ein Verfahren zur Herstellung einer Verbindung der Formel **1** 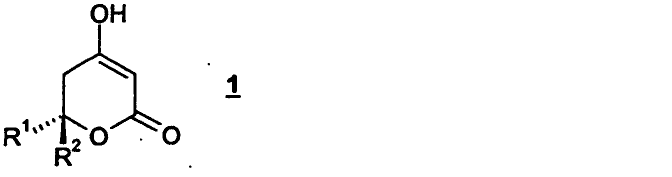 gegebenenfalls in Form ihrer Tautomere,
worin
- R¹ und R²: unabhängig voneinander Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy und CF₃, bedeuten, mit der Maßgabe, daß R¹ und R² nicht gleichzeitig dieselbe Bedeutung aufweisen können,
dadurch gekennzeichnet, daß in einem ersten Schritt eine Verbindung der Formel **1-rac** 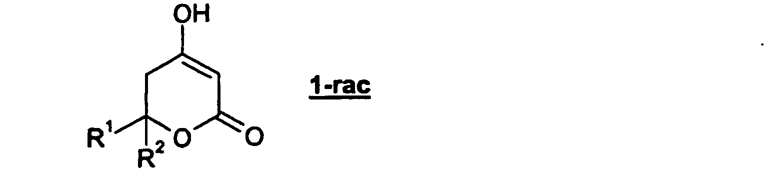 in der R¹ und R² die vorstehend genannten Bedeutungen aufweisen können, in einem geeigneten Lösemittel mit einem oder mehreren, bevorzugt einem chiralen Aminoalkohol der Formel **4** 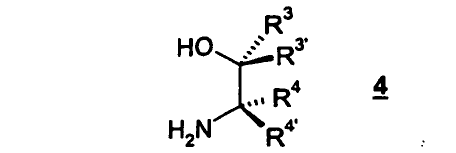 worin
- R³, R^{3'}, R⁴ und R^{4'}: gleich oder verschieden Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy, -S-C₁-C₄-Alkyl, -SO₂-C₁-C₄-Alkyl, NO₂ und CF₃, bedeuten,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt** 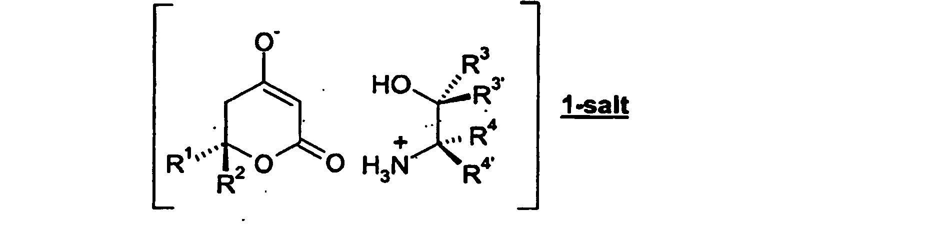 worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung freigesetzt wird.

Erfindungsgemäß bevorzugt ist ein Verfahren zur Herstellung einer Verbindung der Formel **1**, gegebenenfalls in Form ihrer Tautomere,
worin
- R¹ und R²: unabhängig voneinander ein Rest ausgewählt aus der Gruppe bestehend aus Methyl, Ethyl, Propyl, Butyl, Phenyl, Benzyl, Phenylethyl und Phenylpropyl der gegebenenfalls einfach substituiert sein kann durch einen Rest ausgewählt aus der Gruppe bestehend aus Hydroxy, Fluor, Chlor, Brom, Methoxy, Ethyoxy und CF₃
bedeuten, mit der Maßgabe, daß R¹ und R² nicht gleichzeitig dieselbe Bedeutung aufweisen können,
dadurch gekennzeichnet, daß in einem ersten Schritt eine Verbindung der Formel **1-rac** in der R¹ und R² die vorstehend genannten Bedeutungen aufweisen können, in einem geeigneten Lösemittel mit einem oder mehreren, bevorzugt einem chiralen Aminoalkohol der Formel **4**
worin
- R³, R^{3'}, R⁴ und R⁴': gleich oder verschieden Wasserstoff oder ein Rest ausgewählt aus der Gruppe bestehend aus Methyl, Ethyl, Propyl, Phenyl, Benzyl und Phenylethyl, der gegebenenfalls ein- oder zweifach substituiert sein kann durch einen oder zwei Reste ausgewählt aus der Gruppe bestehend aus Hydroxy, Fluor, Chlor, Brom, Methoxy, Ethyoxy, Me-S-, Me-SO₂-, Et-S-, Et-SO₂-, NO₂ und CF₃, bedeuten,
mit der Maßgabe, daß wenn R³ und R³' dieselbe Bedeutung aufweisen, die Reste R⁴ und R⁴' nicht dieselbe Bedeutung aufweisen können, sowie mit der Maßgabe, daß wenn R⁴ und R^{4'} dieselbe Bedeutung aufweisen, die Reste R³ und R³' nicht dieselbe Bedeutung aufweisen können,
zu einem Salz der Formel **1-salt,** worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung 1 freigesetzt wird.

Erfindungsgemäß besonders bevorzugt ist ein Verfahren zur Herstellung einer Verbindung der Formel **1** gegebenenfalls in Form ihrer Tautomere,
worin
- R¹ und R²: unabhängig voneinander ein Rest ausgewählt aus der Gruppe bestehend aus Methyl, Ethyl, Propyl, Butyl, Phenyl, Benzyl, Phenylethyl und Phenylpropyl,
bedeuten, mit der Maßgabe, daß R¹ und R² nicht gleichzeitig dieselbe Bedeutung aufweisen können,
dadurch gekennzeichnet, daß in einem ersten Schritt eine Verbindung der Formel 1-rac, in der R¹ und R² die vorstehend genannten Bedeutungen aufweisen können, in einem geeigneten Lösemittel mit einem oder mehreren, bevorzugt einem chiralen Aminoalkohol der Formel **4**
worin
- R³, R^{3'}, R⁴ und R^{4'}: gleich oder verschieden Wasserstoff oder ein Rest ausgewählt aus der Gruppe bestehend aus Methyl, Ethyl, Propyl, Phenyl und Benzyl, der gegebenenfalls einfach substituiert sein kann durch einen Rest ausgewählt aus der Gruppe bestehend aus Hydroxy, Methoxy, Me-S-, Me-SO₂- und NO₂, bedeuten,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt****,** worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung **1** freigesetzt wird.

Ferner bevorzugt ist ein Verfahren zur Herstellung einer Verbindung der Formel **1** gegebenenfalls in Form ihrer Tautomere, worin
- R¹ und R²: unabhängig voneinander ein Rest ausgewählt aus der Gruppe bestehend aus Ethyl, Propyl, Butyl, Benzyl, Phenylethyl und Phenylpropyl, bedeuten, mit der Maßgabe, daß R¹ und R² nicht gleichzeitig dieselbe Bedeutung aufweisen können,
dadurch gekennzeichnet, daß in einem ersten Schritt eine Verbindung der Formel **1-rac****,** in der R¹ und R² die vorstehend genannten Bedeutungen aufweisen können, in einem geeigneten Lösemittel mit einem oder mehreren, bevorzugt einem chiralen Aminoalkohol der Formel **4**, worin
- R³ und R^{3'}: gleich oder verschieden Wasserstoff oder ein Rest ausgewählt aus der Gruppe bestehend aus Phenyl, Hydroxyphenyl, Methylthiophenyl und Nitrophenyl,
und
- R⁴ und R^{4'}: gleich oder verschieden Wasserstoff oder ein Rest ausgewählt aus der Gruppe bestehend aus Methyl, Hydroxymethyl und Phenyl,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt** , worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung **1** freigesetzt wird.

Erfindungsgemäß besonders bevorzugt ist ein Verfahren zur Herstellung einer Verbindung der Formel **1****,** gegebenenfalls in Form ihrer Tautomere,
worin R¹ Phenylethyl und R² Propyl bedeuten
dadurch gekennzeichnet, daß in einem ersten Schritt eine Verbindung der Formel **1-rac** **,** in der R¹ und R² die vorstehend genannten Bedeutungen aufweisen können, in einem geeigneten Lösemittel mit einem oder mehreren, bevorzugt einem chiralen Aminoalkohol der Formel **4**
worin
- R³ und R^{3'}: gleich oder verschieden Wasserstoff oder ein Rest ausgewählt aus der Gruppe bestehend aus Phenyl, Methylthiophenyl und Nitrophenyl,
und
- R⁴ und R⁴': gleich oder verschieden Wasserstoff oder ein Rest ausgewählt aus der Gruppe bestehend aus Methyl, Hydroxymethyl und Phenyl,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt****,** worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung 1 freigesetzt wird.

Zur Durchführung des erfindungsgemäßen Verfahrens kann wie folgt vorgegangen werden. Für die Darstellung von **1-salt** wird das racemische Gemisch **1-rac** in einem geeigneten organischen Lösemittel, bevorzugt in einem wasserfreien organischen Lösemittel, besonders bevorzugt in einem unpolaren organischen Lösemittel gelöst. Erfindungsgemäß bevorzugt kommen als Lösemittel Acetonitril, Propionitril, Butyronitril, Methylenchlorid, Chloroform, Methylacetat, Ethylacetat, n-Butylacetat, tert-Butylmethylether, iso-Propanol, Tetrahydrofuran, Dioxan, Methanol oder Gemische davon, besonders bevorzugt Acetonitril, Propionitril, Butyronitril, Methylenchlorid, n-Butylacetat oder Gemische davon, besonders bevorzugt Acetonitril, Propionitril, Butyronitril oder Gemische davon in Betracht.

Bei einer Temperatur von -10°C bis 50°C, bevorzugt 0°C bis 40°C, besonders bevorzugt 10-30°C, höchst bevorzugt 20-25°C wird das chirale Amin **4** zugegeben und die erhaltene Mischung über einen Zeitraum von 1-12 h, bevorzugt 3-8 h, besonders bevorzugt 4-6 h bei gleichbleibender Temperatur gerührt gut durchmischt. Pro Mol eingesetztes **1-rac** werden zwischen 0,3 - 2,0 Mol, bevorzugt 0,4 - 1,0 Mol, besonders bevorzugt etwa 0,45 - 0,55 Mol Amin **4** eingesetzt. Gegebenenfalls kann es hilfreich sein, zur Beschleunigung der Kristallisation Impfkristalle zuzusetzen oder sonstige im Stand der Technik bekannte Methoden zur Steigerung der Kristallisationsneigung durchzuführen.
Anschließend wird die Reaktionsmischung auf eine Temperatur von -40°C bis 30°C, bevorzugt -20°C bis 20°C, besonders bevorzugt -10°C bis 15°C, höchst bevorzugt 0°C bis 10°C gebracht und das kristalline **1-salt** abgetrennt. Aus der verbleibenden Lösung wird unter analoger Anwendung von im Stand der Technik bekannten Methoden das zu **1-salt** diastereomere Salz zugänglich.
Die so erhaltenen Kristalle werden anschließend mit einem der vorstehend genannten organischen Lösemittel, bevorzugt mit dem organichen Lösemittel, welches zur Durchführung der Reaktion verwendet wird, gewaschen und im Vakuum getrocknet.

Zur Freisetzung der Verbindungen der Formel **1** aus den Salzen **1-salt** kann erfindungsgemäß wie folgt vorgegangen werden. Die Verbindungen **1-salt** werden hierzu in einem geeigneten Lösemittel, vorzugsweise in einem polaren Lösemittel, besondres bevorzugt in Wasser aufgenommen. Hierzu wird ein organisches Lösemittel, bevorzugt ein organisches, mit Wasser nicht mischbares Lösemittel gegeben. Dieses Lösemittel ist bevorzugt ausgewählt aus der Gruppe bestehend aus Toluol, Methyltert.-Butylether, Methylenchlorid, Chloroform, Methylacetat, Ethylacetat, n-Butylacetat und Gemischen davon, besonders bevorzugt ausgewählt aus Toluol, Methyltert.-Butylether, Ethylacetat, n-Butylacetat und Gemischen davon, wobei Toluol und Methylter.-Butylether eine besondere Bedeutung zukommt. Das Verhältnis in dem das vorstehend genannte polare Lösemittel und das vorstehend genannte nicht mit Wasser mischbare Lösemittel zum einsatz gelangen liegt erfindungsgemäß in einem Bereich von 1:10 (v/v) bis 10:1 (v/v), bevorzugt 1:5 (v/v) bis 5:1 (v/v), besonders bevorzugt 3:1 (v/v) bis 1:3 (v/v). Zu dieser Mischung wird anschließend eine organische oder anorganische Säure zugesetzt, wobei die Verwendung anorganischer Mineralsäuren erfindungsgemäß bevorzugt ist. Bevorzugt gelangen als anorganischen Säuren die folgenden Säuren zur Anwendung: Salzsäure, Schwefelsäure, Salpetersäure, Ameisensäure, Essigsäure oder Phosphoraäure, bevorzugt Salzsäure, Schwefelsäure oder Phosphorsäure, besonders bevorzugt Salzsäure und Schwefelsäure, wobei der Schwefelsäure zur Durchführung dieses Verfahrensschritts erfindungsgemäß besondere Bedeutung zukommt. Die Säure kann in verdünnter oder konzentrierter Form, in From wässriger Lösungen oder in organischen Lösemitteln gelöst, gegebenenfalls auch in gasförmiger Form, soweit technisch durchführbar, erfolgen. Gelangt Schwefelsäure zum Einsatz kann beispielsweise 30%-ige Schwefelsäure (aq) Verwendung finden. Es ist für den Fachmann klar ersichtlich, daß wenigstens stöchiometrische Mengen an Säure zur vollständigen Freisetzung von **1** aus **1-salt** erforderlich sind. Erfindungsgemäß bevorzugt ist allerdings die Säurezugabe im Überschuß. Hierdurch wird erfindungsgemäß ein pH von <5, vorzugsweise ein pH von 0,5-3, besonders bevorzugt ein pH von etwa 1-2 eingestellt.
Nach beendeter Säurezugabe wird zwischen 0,25 - 5 h, bevorzugt 0.5-3 h, besonders bevorzugt 0,75-1,5 h gerührt. Dabei bilden sich zwei Phasen. Die wässrige Phase wird abgetrennt und die verbleibende organische Phase gegebenenfalls noch ein- bis fünfmal mit Wasser gewaschen und abschließend das Lösemittel im Vakuum abdestilliert. Eine weitergehende Reinigung der Produkte ist in der Regel nicht erforderlich. Je nach Kristallisationsneigung von **1** im jeweiligen Lösemittel kann die Isolierung der Zielverbindungen auch mittels Kristallisation erfolgen. Durch Aufarbeitung der organischen Phase, beispielsweise durch Basenzusatz, sind die eingangs in die Synthese eingesetzten Amine **4** wieder erhältlich und können einer erneuten Umsetzung mit **1-rac** wieder zugeführt werden.

Wie aus vorstehender Beschreibung des erfindungsgemäßen Verfahren ersichtlich, kommt den Verbindungen der Formel **1-salt** eine besondere Bedeutung zu, die beispielsweise in der überraschend hohen Kristallisationsneigung dieser Salze begründet liegt.

Dementsprechend betrifft ein weiterer Aspekt der vorliegenden Erfindung die Verbindungen der Formel **1-salt** 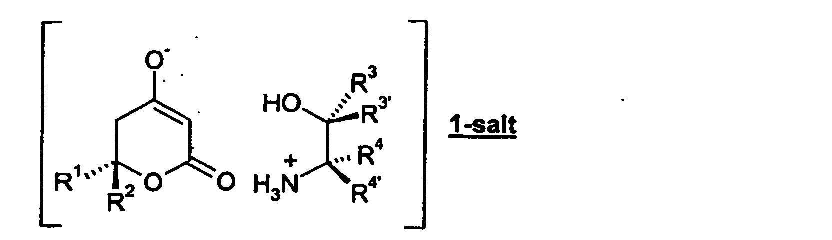 worin
- R¹ und R²: unabhängig voneinander Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy und CF₃;
- R³, R^{3'}, R⁴ und R^{4'}: gleich oder verschieden Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy, -S-C₁-C₄-Alkyl, -SO₂-C₁-C₄-Alkyl, NO₂ und CF₃,
bedeuten, gegebenenfalls in Form ihrer Tautomere,
mit der Maßgabe, daß R¹ und R² nicht gleichzeitig dieselbe Bedeutung aufweisen können,
mit der Maßgabe, daß wenn R³ und R^{3'} dieselbe Bedeutung aufweisen, die Reste R⁴ und R^{4'} nicht dieselbe Bedeutung aufweisen können, sowie
mit der Maßgabe, daß wenn R⁴ und R^{4'} dieselbe Bedeutung aufweisen, die Reste R³ und R^{3'} nicht dieselbe Bedeutung aufweisen können.

Bevorzugt sind die verbindungen der Formel **1-salt**
worin
- R¹ und R²: unabhängig voneinander ein Rest ausgewählt aus der Gruppe bestehend aus Methyl, Ethyl, Propyl, Butyl, Phenyl, Benzyl, Phenylethyl und Phenylpropyl der gegebenenfalls einfach substituiert sein kann durch einen Rest ausgewählt aus der Gruppe bestehend aus Hydroxy, Fluor, Chlor, Brom, Methoxy, Ethyoxy und CF₃;
- R³, R^{3'}, R⁴ und R^{4'}: gleich oder verschieden Wasserstoff oder ein Rest ausgewählt aus der Gruppe bestehend aus Methyl, Ethyl, Propyl, Phenyl, Benzyl und Phenylethyl, der gegebenenfalls ein- oder zweifach substituiert sein kann durch einen oder zwei Reste ausgewählt aus der Gruppe bestehend aus Hydroxy, Fluor, Chlor, Brom, Methoxy, Ethyoxy, Me-S-, Me-SO₂-, Et-S-, Et-SO₂-, NO₂ und CF₃,
bedeuten, gegebenenfalls in Form ihrer Tautomere,
mit der Maßgabe, daß R¹ und R² nicht gleichzeitig dieselbe Bedeutung aufweisen können,
mit der Maßgabe, daß wenn R³ und R^{3'} dieselbe Bedeutung aufweisen, die Reste R⁴ und R^{4'} nicht dieselbe Bedeutung aufweisen können; sowie
mit der Maßgabe, daß wenn R⁴ und R^{4'} dieselbe Bedeutung aufweisen, die Reste R³ und R^{3'} nicht dieselbe Bedeutung aufweisen können.

Besonders bevorzugt sind Verbindungen der Formel **1-salt**
worin
- R¹ und R²: unabhängig voneinander ein Rest ausgewählt aus der Gruppe bestehend aus Methyl, Ethyl, Propyl, Butyl, Phenyl, Benzyl, Phenylethyl und Phenylpropyl,
- R³, R^{3'}, R⁴ und R^{4'}: gleich oder verschieden Wasserstoff oder ein Rest ausgewählt aus der Gruppe bestehend aus Methyl, Ethyl, Propyl, Phenyl und Benzyl, der gegebenenfalls einfach substituiert sein kann durch einen Rest ausgewählt aus der Gruppe bestehend aus Hydroxy, Methoxy, Me-S-, Me-SO₂- und NO₂,
bedeuten, gegebenenfalls in Form ihrer Tautomere,
mit der Maßgabe, daß R¹ und R² nicht gleichzeitig dieselbe Bedeutung aufweisen können,
mit der Maßgabe, daß wenn R³ und R^{3'} dieselbe Bedeutung aufweisen, die Reste R⁴ und R^{4'} nicht dieselbe Bedeutung aufweisen können, sowie
mit der Maßgabe, daß wenn R⁴ und R^{4'} dieselbe Bedeutung aufweisen, die Reste R³ und R^{3'} nicht dieselbe Bedeutung aufweisen können.

Ferner bevorzugt sind Verbindungen der Formel **1-salt**
worin
- R¹ und R²: unabhängig voneinander ein Rest ausgewählt aus der Gruppe bestehend aus Ethyl, Propyl, Butyl, Benzyl, Phenylethyl und Phenylpropyl,
- R³ und R^{3'}: gleich oder verschieden Wasserstoff oder ein Rest ausgewählt aus der Gruppe bestehend aus Phenyl, Hydroxyphenyl, Methylthiophenyl und Nitrophenyl;
- R⁴ und R⁴': gleich oder verschieden Wasserstoff oder ein Rest ausgewählt aus der Gruppe bestehend aus Methyl, Hydroxymethyl und Phenyl,
bedeuten, gegebenenfalls in Form ihrer Tautomere,
mit der Maßgabe, daß R¹ und R² nicht gleichzeitig dieselbe Bedeutung aufweisen können,
mit der Maßgabe, daß wenn R³ und R^{3'} dieselbe Bedeutung aufweisen, die Reste R⁴ und R⁴' nicht dieselbe Bedeutung aufweisen können, sowie
mit der Maßgabe, daß wenn R⁴ und R^{4'} dieselbe Bedeutung aufweisen, die Reste R³ und R³' nicht dieselbe Bedeutung aufweisen können.

Erfindungsgemäß besonders bevorzugt sind ferner Verbindung der Formel **1-salt**
worin
- R¹: Phenylethyl und R² Propyl,
- R³ und R^{3'}: gleich oder verschieden Wasserstoff oder ein Rest ausgewählt aus der Gruppe bestehend aus Phenyl, Methylthiophenyl und Nitrophenyl,
und
- R⁴ und R^{4'}: gleich oder verschieden Wasserstoff oder ein Rest ausgewählt aus der Gruppe bestehend aus Methyl, Hydroxymethyl und Phenyl, gegebenenfalls in Form ihrer Tautomere, mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können.

Aufgrund der zentralen Bedeutung der Verbindungen der Formel **1-salt** als Ausgangsverbindungen für die Synthese optisch aktiver, pharmazeutisch wirskamer Verbindungen, betrifft ein weiterer Aspekt, die Verwendung von Verbindungen der allgemeinen Formel **1-salt** zur Herstellung pharmazeutisch wirksamer Verbindungen. Bevorzugt betrifft die vorliegende Erfindung die Verwendung von Verbindungen der Formel **1-salt** zur Herstellung von Tipranavir.

Ferner betrifft die vorliegende Erfindung Verfahren zur Herstellung pharmazeutisch wirksamer Verbindungen, die dadurch gekennzeichnet sind, daß Verbindungen der Formel **1-salt** eingesetzt werden, die gemäß dem erfindungsgemäßen Verfahren zur Herstellung von **1-salt** erhältlich sind.
Bevorzugt betrifft die vorliegende Erfindung Verfahren zur Herstellung von Tipranavir, die dadurch gekennzeichnet sind, daß Verbindungen der Formel **1-salt** eingesetzt werden, die gemäß dem erfindungsgemäßen Verfahren zur Herstellung von **1-salt** erhältlich sind.

Ferner betrifft ein Aspekt der vorliegenden Erfindung die Verwendung von Verbindungen der allgemeinen Formel **1****,** die gemäß der vorliegenden Erfindung erhalten werden, zur Herstellung pharmazeutisch wirksamer Verbindungen.
Bevorzugt betrifft die vorliegende Erfindung die Verwendung von Verbindungen der Formel **1**, die gemäß der vorliegenden Erfindung erhalten werden, zur Herstellung von Tipranavir.

Ferner betrifft die vorliegende Erfindung Verfahren zur Herstellung pharmazeutisch wirksamer Verbindungen, die dadurch gekennzeichnet sind, daß Verbindungen der Formel **1** eingesetzt werden, die gemäß dem erfindungsgemäßen Verfahren zur Herstellung von **1** erhalten werden.
Bevorzugt betrifft die vorliegende Erfindung Verfahren zur Herstellung von Tipranavir, die dadurch gekennzeichnet sind, daß Verbindungen der Formel **1** eingesetzt werden, die gemäß dem erfindungsgemäßen Verfahren zur Herstellung von **1** erhalten werden.

Der synthetische Zugang zu den Verbindungen **1-salt** erfolgt erfindungsgemäß ausgehend von den Verbindungen der Formel **1-rac****.** Letztere sind nach der in Schema 3 skizzierten Vorgehensweise erhältlich.

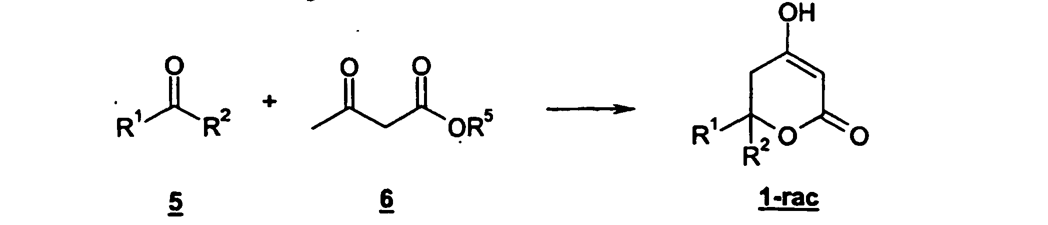

### Schema 3:

Hierzu wird eine geeignet substituierte Carbonylverbindung **5**, in der die Reste R¹ und R² die vorstehend genannten Bedeutungen haben können, mit einem Acetessigsäurederivat **6**, in dem R⁵ C₁-C₄-Alkyl oder für ein Kation ausgewählt aus Lithium, Natrium und Kalium, vorzugsweise Natrium, bevorzugt Methyl, Ethyl, Propyl oder Natrium, besonders bevorzugt Ethyl oder Natrium bedeutet, zur Verbindung **1-rac****,** in der die Reste R¹ und R² die vorstehend genannten Bedeutungen haben können, umgesetzt.

Diese Umsetzung erfolgt in Gegenwart organischer oder anorganischer Basen, bevorzugt in Gegenwart organischer, sterisch anspruchsvoller Basen. Im Rahmen der vorliegenden Erfindung gelangen vorzugsweise solche organischen Basen in Betracht, die ausgewählt sind aus der Gruppe bestehend aus DBU oder DBN. Als sterisch anspruchsvolle organische Basen im Sinne der vorliegenden Erfindung werden ferner verstanden organometallische Verbindungen wie beispielsweise Lithium-, Natrium- und Kaliumsalze sekundärer Amine. Bevorzugte Basen dieses Typs sind ausgewählt aus der Gruppe bestehend aus Lithiumdiisopropylamin, Lithiumdiethylamin, Lithiumhexamethyldisilazan, Natriumhexamethyldisilazan, Kaliumhexamethyldisilazan oder Lithiumtert.-Bitylat, bevorzugt Lithiumdiisopropylamin, Lithiumdiethylamin, Lithiumhexamethyldisilazan, Natriumhexamethyldisilazan, Kaliumhexamethyldisilazan, besonders bevorzugt Lithiumdiisopropylamin, und Lithiumdiethylamin. Letztgenannte Basen sind entweder kommerziell erhältlich oder können nach im Stand der Technik bekannten Verfahren synthetisiert werden.

Zur erfindungsgemäßen Darstellung der Verbindungen der Formel **1-rac** wird eine der vorstehend genannten Basen, die entweder in situ generiert oder direkt eingesetzt wird, in einem geeigneten organischen Lösemittel, bevorzugt in einem organischen wasserfreien Lösemittel vorgelegt. Als Lösemittel kommen vorzugsweise etherische Lösemittel, wie Tetrahydrofuran (THF), Methylethylether, Diethylether, Dioxan oder andere unpolare organische Lösemittel wie Toluol, Hexan oder Heptan in Betracht. Gegebenenfalls können die etherischen Lösemittel auch um Gemisch mit vorstehend genannten unpolaren Lösemitteln Verwendung finden. Die Verwendung der vorstehend genannten etherischen Lösemittel ist hierbei bevorzugt. Besondere Bedeutung kommt den Lösemitteln THF, werden Gemische eingesetzte vorzugsweise THF/Hexan oder THF/Toluol zu.

Die so erhaltene Lösung wird abgekühlt, vorzugsweise auf eine Temperatur von unter 0°C, bevorzugt unter -10°C, besonders bevorzugt unter -20°C. Erfindungsgemäß besonders bevorzugt wird die Reaktion in einem Bereich von -78°C bis -40°C durchgeführt. Sofern eines der vorstehend genannten Lösemittel bei diesen Temperaturbereichen nicht mehr im flüssigen Aggregatszustand vorliegt, bestimmt sich die tiefstmögliche Reaktionstemperatur, wie für den Fachmann ersichtlich, durch den Fließpunkt des verwendeten Lösemittels. Zur gekühlten Lösung der Base in einem der vorstehend genannten Lösemittel wird anschließend das Acetessigsäurederivat **6****,** in dem R⁵ die vorstehend genannten Bedeutungen haben kann, zugegeben. Pro Mol eingesetzte Base werden maximal stöchiometrische Mengen an **6** zugesetzt. Vorzugsweise werden substöchiometrische Mengen an **6** verwendet, wobei das Molverhältnis von **6** zu eingesetzter Base bevorzugt in einem Bereich von 0,9:1 - 0,2:1, besonders bevorzugt 0,7:1 - 0,3:1, besonders bevorzugt 0,6:1 - 0,4:1 liegt.

Nach Zugabe von **6** wird über einen Zeitraum von etwa 5 Minuten bis 1 h bei konstanter Temperatur gerührt und eine Lösung von **5** in einem der vorstehend genannten Lösemittel, vorzugsweise im selben organischen Lösemittel, langsam zugetropft. Pro Mol eingesetzte Verbindung **6** wird maximal 1 Mol **5** verwendet. Vorzugsweise werden substöchiometrische Mengen an **5** verwendet, wobei das Molverhältnis von **5** zu **6** bevorzugt in einem Bereich von 0,9:1 - 0,2:1, besonders bevorzugt 0,8:1 - 0,3:1, besonders bevorzugt 0;7:1 - 0,5:1 liegt. Nach beendeter Zugabe wird entweder bei konstanter oder bei leicht erhöhter Temperatur weitergerührt. Wird die Temperatur erhöht wird sie vorzugsweise dennoch unter 0°C, bevorzugt unter -10°C; besonders bevorzugt unter -20°C gehalten. Erfindungsgemäß besonders bevorzugt wird die Reaktion in einem Bereich von -40C bis -20°C geführt. Die Reaktionsdauer liegt erfindungsgemäß in einem Bereich von 0,5 - 8 h, bevorzugt 1 - 5 h, besonders bevorzugt 1,5 - 3 h.

Der Abbruch der Reaktion kann nach im Stand der Technik bekannten Methoden, beispielsweise durch Zugabe wässriger Lösungen, beispielsweise wässriger Ammoniumchloridlösung, erfolgen. Die Aufarbeitung gelingt ebenfalls in Analogie zu im Stand der Technik bekannten Vorgehensweisen.

Als Alkylgruppen (auch soweit sie Bestandteil anderer Reste sind) werden, soweit nicht anders definiert, verzweigte und unverzweigte Alkylgruppen mit 1 bis 4 Kohlenstoffatomen betrachtet. Als Beispiele seien folgende Kohlenwasserstoffreste genannt: Methyl, Ethyl, Propyl, 1-Methylethyl (Isopropyl), n-Butyl, 1-Methylpropyl, 2-Methylpropyl, 1,1-Dimethylethyl (tert.Butyl). Dabei sind von den Definitionen Propyl und Butyl die jeweiligen isomeren Reste stets mit umfaßt. Gegebenenfalls werden für die vorstehend genannten Alkylgruppen auch die gängigen Abkürzungen Me für Methyl, Et für Ethyl, Prop für Propyl und But für Butyl verwendet.

Als Alkylengruppen werden verzweigte und unverzweigte Alkylenbrücken mit 1 bis 4 Kohlenstoffatomen betrachtet. Beispielsweise werden genannt: Methylen, Ethylen, Propylen und Butylen. Sofern nicht anders genannt, sind von den vorstehend genannten Bezeichnungen Propylen und Butylen sämtliche der möglichen isomeren Formen umfaßt. Dementsprechend umfaßt die Bezeichnung Propylen die isomeren Brücken n-Propylen, Methylethylen und Dimethylmethylen und die Bezeichnung Butylen die isomeren Brücken n-Butylen, 1-Methylpropylen, 2-Methylpropylen, 1.1-Dimethylethylen und 1.2-Dimethylethylen.

Cycloalkyl steht im allgemeinen für einen gesättigten cyclischen Kohlenwasserstoffrest mit 3 bis 8 Kohlenstoffatomen. Bevorzugt sind cyclische Kohlenwasserstoffe mit 3 bis 6 Kohlenstoffatomen. Als Beispiele seien Cyclopropyl, Cyclobutyl, Cyclopentyl, Cyclohexyl, Cycloheptyl und Cyclooctyl genannt..

Alkyloxy, welches gegebenenfalls auch als Alkoxy bezeichnet werden kann, steht im allgemeinen für eine über ein Sauerstoffatom gebundene, geradkettige oder verzweigte Alkylgruppe mit 1 bis 4 Kohlenstoffatmen. Besonders bevorzugt ist die Methoxygruppe.

Der Begriff Aryl steht für ein aromatisches Ringsystem mit 6 bis 10 Kohlenstoffatomen. Bevorzugte Arylreste sind Naphthyl und Phenyl, wobei der Phenylrest besonders bevorzugt ist. Gegebenenfalls werden für Naphthyl die Naph und für Phenyl Ph als Abkürzungen verwendet.

Unter Aryl-alkylen oder Alkylen-Aryl werden im Sinne der Erfindung Arylgruppen verstanden, die über eine Alkylenbrücke verknüpft sind, wobei die vorstehend genannten Definitionen für Alkylengruppen und Arylgruppen zur Anwendung gelangen. Erfindungsgemäß bevorzugte Alkylen-Aryl-Gruppen sind, soweit nicht anders definiert, Benzyl, 2-Phenylethyl und 3-Phenylpropyl.

Halogen steht im Rahmen der vorliegenden Erfindung für Fluor, Chlor, Brom oder lod, wobei Fluor, Chlor und Brom, soweit nicht anders definiert, bevorzugt sind.

Im Rahmen der vorliegenden Erfindung ist eine Bezugnahme auf Verbindungen der Formel **1** als Bezugnahme auf die Verbindung in einer optisch aktiven Form zu verstehen. Die jeweilige optisch aktive Form ergibt sich aus der jeweiligen Definition der Reste R¹ und R². Ein Bezugnahme auf **1** schließt alle enantiomeren Gemische der beiden möglichen Enantiomere von **1** mit ein, die nicht racemisch sind. Racemische Gemische werden, wie bereits vorstehend definiert, als **1-rac** bezeichnet. Ein Bezugnahme auf **1** schließt ferner die jeweiligen Tautomeren Formen von **1** mit ein.

Im Rahmen der vorliegenden Erfindung ist eine Bezugnahme auf Verbindungen der Formel **1-salt** als Bezugnahme auf die Verbindung in einer optisch aktiven Form zu verstehen. Die jeweilige optisch aktive Form ergibt sich aus der jeweiligen Definition der Reste R¹, R², R³, R^{3'}, R⁴ und R^{4'}. Ein Bezugnahme auf **1-salt** schließt alle diastereomeren Gemische der möglichen Diastereomere von **1-salt** mit ein, die nicht racemisch sind. Ein Bezugnahme auf **1-salt** schließt ferner die jeweiligen Tautomeren Formen von **1-salt** mit ein.

Die nachfolgenden Beispiele dienen der Illustration exemplarisch durchgeführter Syntheseverfahren zur Herstellung von Verbindungen der Formel **1**.

### Darstellung der Verbindung der Formel 1-rac

(in der R¹ 2-Phenylethyl und R² n-Propyl bedeutet)
104 ml (1,00 mol) Diethylamin in 160 ml THF werden auf -50°C gekühlt. Zu dieser Lösung werden innerhalb von 10 min 400 ml (1,00 mol) einer 2.5 M n-BuLi-Lösung in n-Hexan gegeben. Nach beendeter Zugabe wird weitere 10 min nachgerührt und anschließend 63 ml (0,500 mol) Acetessigester zugegeben. Nach 10 min werden 60 g (0,340 mol) 1-Phenylhexan-3-on in 80 ml THF in 30 min bei -50 °C zugetropft. Anschließend wird auf -30 °C erwärmt und weiter 2 h gerührt. Die Reaktionslösung wird zu 1200 ml ges. NH₄Cl-Lösung gegeben, die wässrige Phase abgetrennt und die organische Phase mit 400 ml ges. NH₄Cl-Lösung und mit 200 ml 2N HCl gewaschen. Anschließend wird die organische Phase mit NaHCO3-Lsg neutral gewaschen, abgetrennt und das Lösemittel im Vakuum abdestilliert. Das erhaltene Öl (118 g) wird bei Raumtemperatur mit einer Lösung aus 29,4 g (1,20 mol) KOH (85%ig) in 140 ml MeOH versetzt. Nach beendeter Reaktion (ca. 10h) wird vom MeOH abdestilliert. Den Rückstand wird in 400 ml Wasser und 400 ml Toluol aufgenommen und die wässrige Phase nach Phasentrennung nochmals mit 400 ml Toluol extrahiert. Die wässrige Phase wird mit 136 ml 30 % iger H₂SO₄ auf pH < 2 gestellt und mit 400 ml Toluol extrahiert.. Die Toluolphase wird dreimal mit 200 ml Wasser nachgewaschen. Das Toluol wird ca. bis auf die Hälfte des Volumens eingeengt. Der Rückstand wird mit 500 ml Octan versetzt und bis zur Kristallisation gerührt. Man erhält 67,5 g (76%) der Verbindung **1-rac**.

### Darstellung der Verbindung der Formel 1-salt

(in der R¹ 2-Phenylethyl, R² n-Propyl, R³ Wasserstoff, R³' 4-Methylthiophenyl, R⁴ Hydroxymethyl und R⁴' Wasserstoff bedeutet)
29 g (110 mmol) der nach vorstehendem Verfahren erhaltenen Verbindung **1-rac** werden in 250 ml Acetonitril gelöst. Nach Zugabe von 10,6 g (50,0 mmol) (+)-Thiomicamin **4** bildet sich eine Lösung. Man rührt 5 Stunden bei Raumtemperatur, kühlt auf ca 5 °C ab und saugt die Kristalle über einen Büchnertrichter ab. Die erhaltenen Kristalle werden mit 150 ml Acetonitril gewaschen. Die Kristalle werden im Vakuumtrockenschrank 12 h getrocknet. Man erhält 21,3 g (90% Ausbeute bezogen auf eingesetztes Amin **4)** farblose Kristalle. Das Diastereomerenverhältnis beträgt ohne weiteren Kristallisatiosnschritt bereits etwa 90:10. Fall erforderlich kann die optische Reinheit durch einen erneuten Kristallisationsschritt weiter erhöht werden.

### Darstellung der Verbindung der Formel 1

(in der R¹ 2-Phenylethyl und R² n-Propyl bedeutet)
29 g (110 mmol) der gemäß vorstehend beschriebener Vorgehensweise erhaltenen Verbindung **1-salt** werden in 290 ml Wasser mit 290 Methyl-tert.-Butylether versetzt. Dazu werden 15 ml 30%ige Schwefelsäure gegeben (pH =1,5). Nach einer Stunde Rühren bilden sich zwei Phasen. Die wässrige Phase wird abgetrennt. Die organische Phase wird dreimal mit je 100 ml Wasser gewaschen. Anschließend wird die organische Phase im Vakuum bis zum Rückstand eingedampft.
Man erhält 14,0 g (Ausbeute 88%) der Verbindung **1.**

Tabelle 1 faßt die Verbindungen der Formel **1-salt** , die nach der erfindungsgemäßen Vorgehensweise erhalten wurden, zusammen.

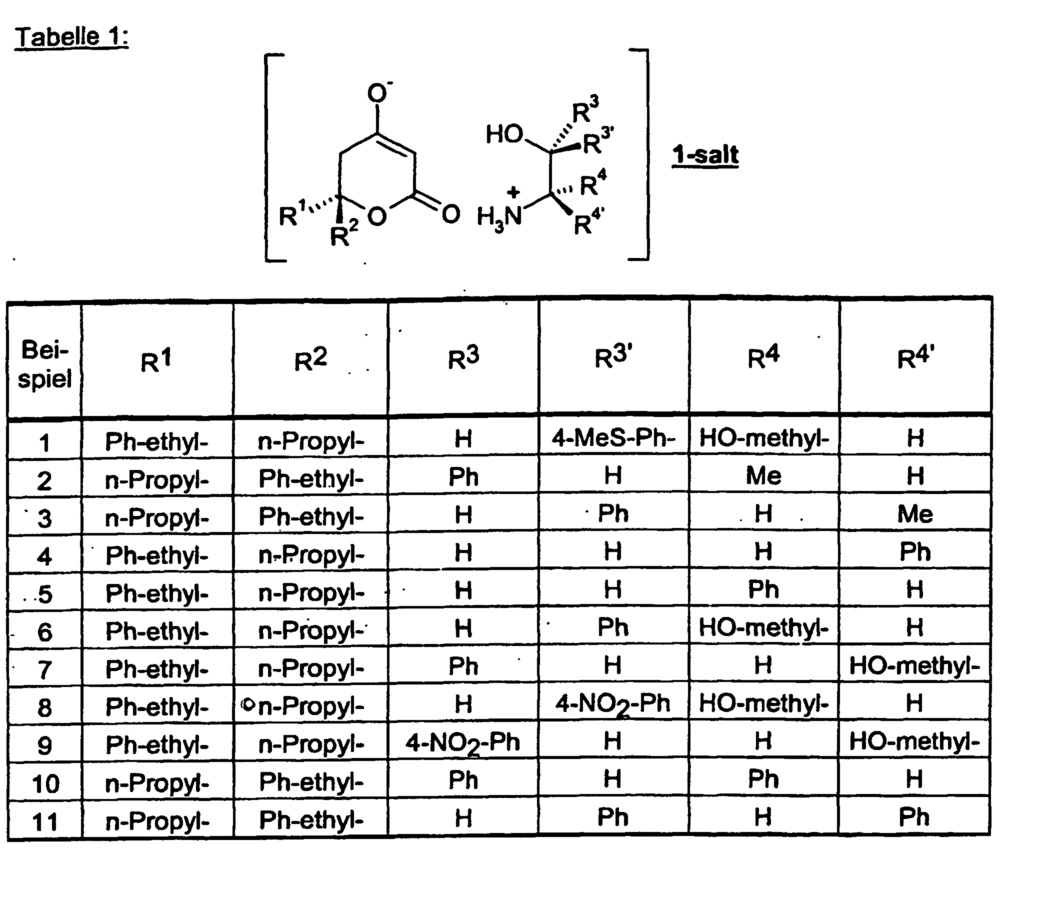

## Patentansprüche

1. Verfahren zur Herstellung einer Verbindung der Formel **1** 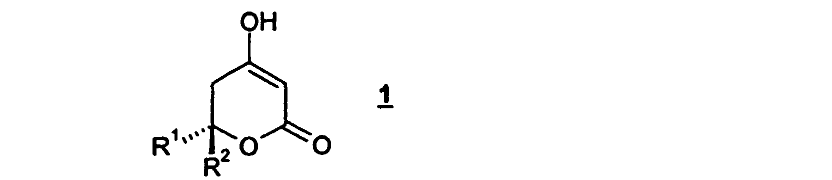 gegebenenfalls in Form ihrer Tautomere,
worin
R¹ und R² unabhängig voneinander Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy und CF₃, bedeuten, mit der Maßgabe, daß R¹ und R² nicht gleichzeitig dieselbe Bedeutung aufweisen können,
**dadurch gekennzeichnet, daß** in einem ersten Schritt eine Verbindung der Formel **1-rac** 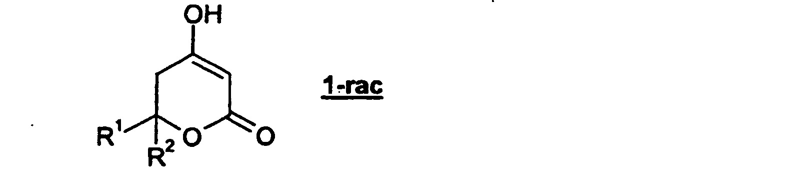 in der R¹ und R² die vorstehend genannten Bedeutungen aufweisen können, in einem geeigneten Lösemittel mit einem oder mehreren, bevorzugt einem chiralen Aminoalkohol der Formel **4** 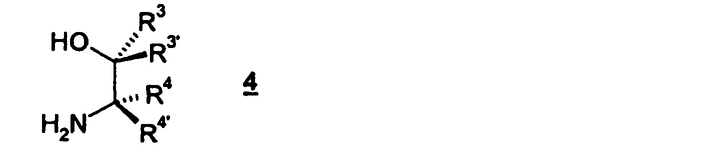 worin
R³, R^{3'}, R⁴ und R^{4'} gleich oder verschieden Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy, -S-C₁-C₄-Alkyl, -SO₂-C₁-C₄-Alkyl, NO₂ und CF₃, bedeuten,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt** 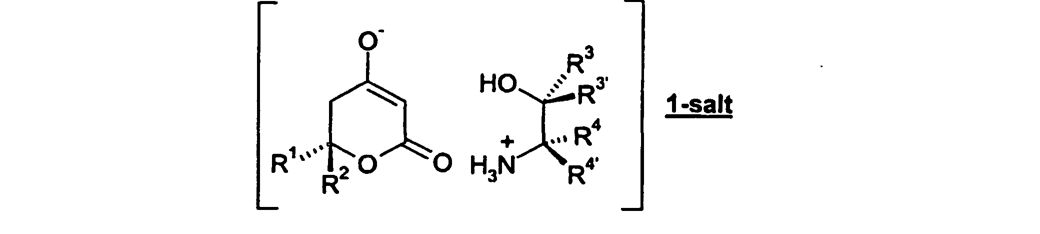 worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung freigesetzt wird.

2. Verfahren zur Herstellung einer Verbindung der Formel **1***,* gegebenenfalls in Form ihrer Tautomere, nach Anspruch 1,
worin
R¹ und R² unabhängig voneinander ein Rest ausgewählt aus der Gruppe bestehend aus Methyl, Ethyl, Propyl, Butyl, Phenyl, Benzyl, Phenylethyl und Phenylpropyl der gegebenenfalls einfach substituiert sein kann durch einen Rest ausgewählt aus der Gruppe bestehend aus Hydroxy, Fluor, Chlor, Brom, Methoxy, Ethyoxy und CF₃
bedeuten, mit der Maßgabe, daß R¹ und R² nicht gleichzeitig dieselbe Bedeutung aufweisen können,
**dadurch gekennzeichnet, daß** in einem ersten Schritt eine Verbindung der Formel **1-rac** in der R¹ und R² die vorstehend genannten Bedeutungen aufweisen können, in einem geeigneten Lösemittel mit einem oder mehreren, bevorzugt einem chiralen Aminoalkohol der Formel **4**
worin
R³, R^{3'}, R⁴ und R^{4'} gleich oder verschieden Wasserstoff oder ein Rest ausgewählt aus der Gruppe bestehend aus Methyl, Ethyl, Propyl, Phenyl, Benzyl und Phenylethyl, der gegebenenfalls ein- oder zweifach substituiert sein kann durch einen oder zwei Reste ausgewählt aus der Gruppe bestehend aus Hydroxy, Fluor, Chlor, Brom, Methoxy, Ethyoxy, Me-S-, Me-SO₂-, Et-S-, Et-SO₂-, NO₂ und CF₃ , bedeuten,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt,** worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung **1** freigesetzt wird.

3. Verfahren zur Herstellung einer Verbindung der Formel **1**, gegebenenfalls in Form ihrer Tautomere, nach einem der Ansprüche 1 oder 2,
worin
R¹ und R² unabhängig voneinander ein Rest ausgewählt aus der Gruppe bestehend aus Methyl, Ethyl, Propyl, Butyl, Phenyl, Benzyl, Phenylethyl und Phenylpropyl,
bedeuten, mit der Maßgabe, daß R¹ und R² nicht gleichzeitig dieselbe Bedeutung aufweisen können,
**dadurch gekennzeichnet, daß** in einem ersten Schritt eine Verbindung der Formel **1-rac****,** in der R¹ und R² die vorstehend genannten Bedeutungen aufweisen können, in einem geeigneten Lösemittel mit einem oder mehreren, bevorzugt einem chiralen Aminoalkohol der Formel **4**
worin
R³, R^{3'}, R⁴ und R^{4'} gleich oder verschieden Wasserstoff oder ein Rest ausgewählt aus der Gruppe bestehend aus Methyl, Ethyl, Propyl, Phenyl und Benzyl, der gegebenenfalls einfach substituiert sein kann durch einen Rest ausgewählt aus der Gruppe bestehend aus Hydroxy, Methoxy, Me-S-, Me-SO₂- und NO₂, bedeuten,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt**, worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung **1** freigesetzt wird.

4. Verfahren zur Herstellung einer Verbindung der Formel **1****,** gegebenenfalls in Form ihrer Tautomere, nach einem der Ansprüche 1, 2 oder 3,
worin
R¹ und R² unabhängig voneinander ein Rest ausgewählt aus der Gruppe bestehend aus Ethyl, Propyl, Butyl, Benzyl, Phenylethyl und Phenylpropyl, bedeuten, mit der Maßgabe, daß R¹ und R² nicht gleichzeitig dieselbe Bedeutung aufweisen können,
**dadurch gekennzeichnet, daß** in einem ersten Schritt eine Verbindung der Formel **1-rac****,** in der R¹ und R² die vorstehend genannten Bedeutungen aufweisen können, in einem geeigneten Lösemittel mit einem oder mehreren, bevorzugt einem chiralen Aminoalkohol der Formel **4**, worin
R³ und R^{3'} gleich oder verschieden Wasserstoff oder ein Rest ausgewählt aus der Gruppe bestehend aus Phenyl, Hydroxyphenyl, Methylthiophenyl und Nitrophenyl,
und
R⁴ und R^{4'} gleich oder verschieden Wasserstoff oder ein Rest ausgewählt aus der Gruppe bestehend aus Methyl, Hydroxymethyl und Phenyl,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können,
zu einem Salz der Formel **1-salt** , worin die Reste R¹, R², R³, R^{3'}, R⁴ und R^{4'} die vorstehend genannten. Bedeutungen haben können, umgesetzt wird, die erhaltene kristalline Verbindung **1-salt** isoliert und daraus die Zielverbindung **1** freigesetzt wird.

5. Verfahren zur Herstellung einer Verbindung der Formel **1**, gegebenenfalls in Form ihrer Tautomere, nach einem der Ansprüche 1, 2, 3 oder 4,
worin
R¹ Phenylethyl und R² Propyl bedeuten
**dadurch gekennzeichnet, daß** in einem ersten Schritt eine Verbindung der Formel **1-rac** , in der R¹ und R² die vorstehend genannten Bedeutungen aufweisen können, in einem geeigneten Lösemittel mit einem oder mehreren, bevorzugt einem chiralen Aminoalkohol der Formel **4**
worin
R³ und R^{3'} gleich oder verschieden Wasserstoff oder ein Rest ausgewählt aus der Gruppe bestehend aus Phenyl, Methylthiophenyl und Nitrophenyl,
und
R⁴ und R^{4'} gleich oder verschieden Wasserstoff oder ein Rest ausgewählt aus der Gruppe bestehend aus Methyl, Hydroxymethyl und Phenyl,
mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R⁴' dieselbe Bedeutung aufweisen, die Reste R³ und R^{3'} nicht dieselbe Bedeutung aufweisen können,
zu einem Salz der Formel **1-salt****,** worin die Reste R¹, R², R³, R^{3'}, R⁴ und R^{4'} die vorstehend genannten Bedeutungen haben können, umgesetzt wird, die erhaltene kristalline Verbindung **1-salt** isoliert und daraus die Zielverbindung **1** freigesetzt wird.

6. Verbindungen der Formel **1-salt** 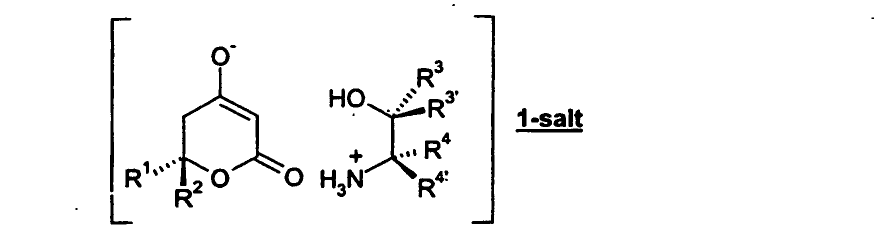 worin
R¹ und R² unabhängig voneinander Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀-Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy und CF₃;
R³, R^{3'}, R⁴ und R^{4'} gleich oder verschieden Wasserstoff oder ein Rest ausgewählt aus der Gruppe bestehend aus C₁-C₄-Alkyl, C₃-C₈-Cycloalkyl, C₆-C₁₀-Aryl und -C₁-C₄-Alkylen-C₆-C₁₀ -Aryl, der gegebenenfalls ein-, zwei- oder dreifach substituiert sein kann durch einen oder mehrere Reste ausgewählt aus der Gruppe bestehend aus Hydroxy, Halogen, C₁-C₄-Alkoxy, -S-C₁-C₄-Alkyl, -SO₂-C₁-C₄-Alkyl, NO₂ und CF₃,
bedeuten, gegebenenfalls in Form ihrer Tautomere,
mit der Maßgabe, daß R¹ und R² nicht gleichzeitig dieselbe Bedeutung aufweisen können, mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können.

7. Verbindungen der Formel **1-salt** gemäß Anspruch 6,
worin
R¹ und R² unabhängig voneinander ein Rest ausgewählt aus der Gruppe bestehend aus Methyl, Ethyl, Propyl, Butyl, Phenyl, Benzyl, Phenylethyl und Phenylpropyl der gegebenenfalls einfach substituiert sein kann durch einen Rest ausgewählt aus der Gruppe bestehend aus Hydroxy, Fluor, Chlor, Brom, Methoxy, Ethyoxy und CF₃;
R³, R^{3'}, R⁴ und R^{4'} gleich oder verschieden Wasserstoff oder ein Rest ausgewählt aus der Gruppe bestehend aus Methyl, Ethyl, Propyl, Phenyl, Benzyl und Phenylethyl, der gegebenenfalls ein- oder zweifach substituiert sein kann durch einen oder zwei Reste ausgewählt aus der Gruppe bestehend aus Hydroxy, Fluor, Chlor, Brom, Methoxy, Ethyoxy, Me-S-, Me-SO₂-, Et-S-, Et-SO₂-, NO₂ und CF₃ ,
bedeuten, gegebenenfalls in Form ihrer Tautomere,
mit der Maßgabe, daß R¹ und R² nicht gleichzeitig dieselbe Bedeutung aufweisen können, mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können.

8. Verbindungen der Formel **1-salt** gemäß einem der Ansprüche 6 oder 7,
worin
R¹ und R² unabhängig voneinander ein Rest ausgewählt aus der Gruppe bestehend aus Methyl, Ethyl, Propyl, Butyl, Phenyl, Benzyl, Phenylethyl und Phenylpropyl,
R³, R^{3'}, R⁴ und R^{4'} gleich oder verschieden Wasserstoff oder ein Rest ausgewählt aus der Gruppe bestehend aus Methyl, Ethyl, Propyl, Phenyl und Benzyl, der gegebenenfalls einfach substituiert sein kann durch einen Rest ausgewählt aus der Gruppe bestehend aus Hydroxy, Methoxy, Me-S-, Me-SO₂- und NO₂,
bedeuten, gegebenenfalls in Form ihrer Tautomere,
mit der Maßgabe, daß R¹ und R² nicht gleichzeitig dieselbe Bedeutung aufweisen können, mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können.

9. Verbindungen der Formel **1-salt** gemäß einem der Anspüche 6, 7 oder 8,
worin
R¹ und R² unabhängig voneinander ein Rest ausgewählt aus der Gruppe bestehend aus Ethyl, Propyl, Butyl, Benzyl, Phenylethyl und Phenylpropyl,
R³ und R^{3'} gleich oder verschieden Wasserstoff oder ein Rest ausgewählt aus der Gruppe bestehend aus Phenyl, Hydroxyphenyl, Methylthiophenyl und Nitrophenyl;
R⁴ und R^{4'} gleich oder verschieden Wasserstoff oder ein Rest ausgewählt aus der Gruppe bestehend aus Methyl, Hydroxymethyl und Phenyl,
bedeuten, gegebenenfalls in Form ihrer Tautomere,
mit der Maßgabe, daß R¹ und R² nicht gleichzeitig dieselbe Bedeutung aufweisen können, mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung ausweisen, die Reste R³ und R^{3'} nicht dieselbe Bedeutung aufweisen können.

10. Verbindung der Formel **1-salt** gemäß einem der Ansprüche 6 bis 9,
worin
R¹ Phenylethyl und R² Propyl,
R³ und R^{3'} gleich oder verschieden Wasserstoff oder ein Rest ausgewählt aus der Gruppe bestehend aus Phenyl, Methylthiophenyl und Nitrophenyl,
und
R⁴ und R^{4'} gleich oder verschieden Wasserstoff oder ein Rest ausgewählt aus der Gruppe bestehend aus Methyl, Hydroxymethyl und Phenyl, gegebenenfalls in Form ihrer Tautomere, mit der Maßgabe, daß wenn R³ und R^{3'} dieselbe Bedeutung aufweisen, die Reste R⁴ und R^{4'} nicht dieselbe Bedeutung aufweisen können, sowie mit der Maßgabe, daß wenn R⁴ und R^{4'} dieselbe Bedeutung aufweisen, die Reste R³ und R^{3'} nicht dieselbe Bedeutung aufweisen können.

11. Verwendung von Verbindungen der allgemeinen Formel **1-salt** gemäß einem der Ansprüche 6 bis 10 zur Herstellung pharmazeutisch wirksamer Verbindungen.

12. Verwendung von Verbindungen der Formel **1-salt** nach Anspruch 11 zur Herstellung von Tipranavir.

## Claims

1. Process for preparing a compound of formula **1** 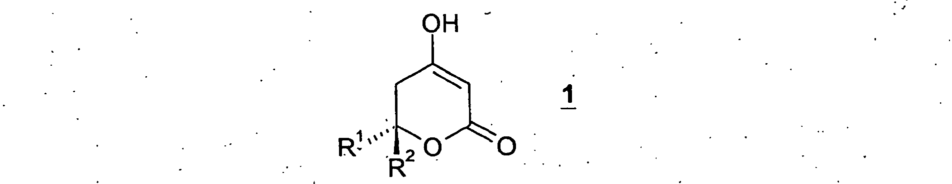 optionally in the form of the tautomers thereof,
wherein
R¹ and R² independently of one another denote hydrogen or a group selected from among C₁-C₄-alkyl, C₃-C₈-cycloalkyl, C₆-C₁₀-aryl and -C₁-C₄-alkylene-C₆-C₁₀-aryl, which may optionally be mono-, di- or trisubstituted by one or more groups selected from among hydroxy, halogen, C₁-C₄-alkoxy and CF₃, with the proviso that R¹ and R² cannot simultaneously have the same meaning,
**characterised in that** in a first step a compound of formula **1-rac** 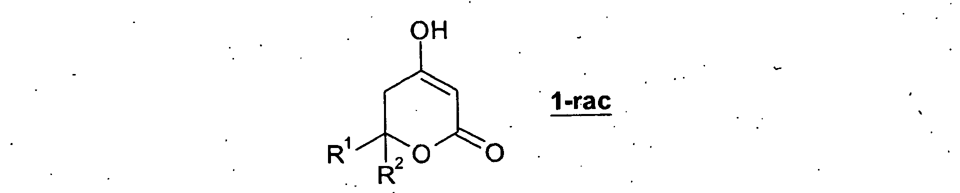 wherein R¹ and R² may have the meanings given hereinbefore,
is reacted in a suitable solvent with one or more, preferably one chiral aminoalcohol of formula **4** 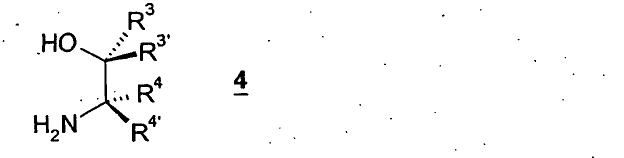 wherein
R³, R^{3'}, R⁴ and R^{4'} which may be identical or different denote hydrogen or a group selected from among C₁-C₄-alkyl, C₃-C₈-cycloalkyl, C₆-C₁₀-aryl and C₁-C₄-alkylene-C₆-C₁₀-aryl, which may optionally be mono-, di- or trisubstituted by one or more groups selected from among hydroxy, halogen, C₁-C₄-alkoxy, -S-C₁-C₄-alkyl, -SO₂-C₁-C₄-alkyl, NO₂ and CF₃,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning,
to form a salt of formula **1-salt** 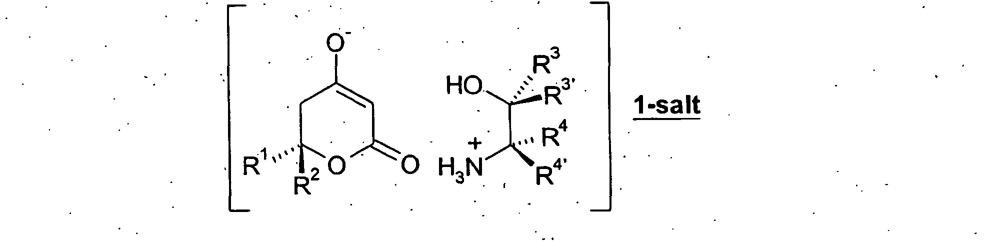 wherein the groups R¹, R², R³, R^{3'}, R⁴ and R^{4'} may have the meanings given hereinbefore, the resulting crystalline compound **1-salt** is isolated and the target compound is liberated therefrom.

2. Process for preparing a compound of formula **1**, optionally in the form of the tautomers thereof, according to claim 1,
wherein
R¹ and R² independently of one another denote a group selected from among methyl, ethyl, propyl, butyl, phenyl, benzyl, phenylethyl and phenylpropyl which may optionally be monosubstituted by a group selected from among hydroxy, fluorine, chlorine, bromine, methoxy, ethoxy and CF₃,
with the proviso that R¹ and R² cannot simultaneously have the same meaning,
**characterised in that** in a first step a compound of formula **1-rac**
wherein R¹ and R² may have the meanings given hereinbefore, is reacted in a suitable solvent with one or more, preferably one chiral aminoalcohol of formula **4**
wherein
R³, R^{3'}, R⁴ and R^{4'} which may be identical or different denote hydrogen or a group selected from among methyl, ethyl, propyl, phenyl, benzyl and phenylethyl, which may optionally be mono- or disubstituted by one or two groups selected from among hydroxy, fluorine, chlorine, bromine, methoxy, ethoxy, Me-S-, Me-SO₂-, Et-S-, Et-SO₂-, NO₂ and CF₃,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning,
to form a salt of formula **1-salt** wherein the groups R¹, R², R³, R^{3'}, R⁴ and R^{4'} may have the meanings given hereinbefore, the resulting crystalline compound **1-salt** is isolated and the target compound 1 is liberated therefrom.

3. Process for preparing a compound of formula **1**, optionally in the form of the tautomers thereof, according to one of claims 1 or 2,
wherein
R¹ and R² independently of one another denote a group selected from among methyl, ethyl, propyl, butyl, phenyl, benzyl, phenylethyl and phenylpropyl,
with the proviso that R¹ and R² cannot simultaneously have the same meaning,
**characterised in that** in a first step a compound of formula **1-rac****,**
wherein R¹ and R² may have the meanings given hereinbefore, is reacted in a suitable solvent with one or more, preferably one chiral aminoalcohol of formula **4**
wherein
R³, R^{3'}, R⁴ and R^{4'} which may be identical or different denote hydrogen or a group selected from among methyl, ethyl, propyl, phenyl and benzyl, which may optionally be monosubstituted by a group selected from among hydroxy, methoxy, Me-S-, Me-SO₂- and NO₂,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning,
to form a salt of formula **1-salt****,** wherein the groups R¹, R², R³, R^{3'}, R⁴ and R^{4'} may have the meanings given hereinbefore, the resulting crystalline compound **1-salt** is isolated and the target compound **1** is liberated therefrom.

4. Process for preparing a compound of formula **1**, optionally in the form of the tautomers thereof, according to one of claims 1, 2 or 3,
wherein
R¹ and R² independently of one another denote a group selected from among ethyl, propyl, butyl, benzyl, phenylethyl and phenylpropyl,
with the proviso that R¹ and R² cannot simultaneously have the same meaning,
**characterised in that** in a first step a compound of formula **1-rac,**
wherein R¹ and R² may have the meanings given hereinbefore,
is reacted in a suitable solvent with one or more, preferably one chiral aminoalcohol of formula **4**, wherein
R³ and R^{3'} which may be identical or different denote hydrogen or a group selected from among phenyl, hydroxyphenyl, methylthiophenyl and nitrophenyl,
and
R⁴ and R^{4'} which may be identical or different denote hydrogen or a group selected from among methyl, hydroxymethyl and phenyl,
with the proviso that if R³ and R^{3'} have the same meaning, the groups R⁴ and R^{4'} cannot have the same meaning, and with the proviso that if R⁴ and R^{4'} have the same meaning, the groups R³ and R^{3'} cannot have the same meaning,
to form a salt of formula **1-salt****,** wherein the groups R¹; R², R³, R^{3'}, R⁴ and R^{4'} may have the meanings given hereinbefore, the resulting crystalline compound **1-salt** is isolated and the target compound **1** is liberated therefrom.

5. Process for preparing a compound of formula **1**, optionally in the form of the tautomers thereof, according to one of claims 1, 2, 3 or 4,
wherein
R¹ denotes phenylethyl and R² denotes propyl,
**characterised in that** in a first step a compound of formula **1-rac****,**
wherein R¹ and R² may have the meanings given hereinbefore, is reacted in a suitable solvent with one or more, preferably one chiral aminoalcohol of formula **4**
wherein
R³ and R^{3'} which may be identical or different denote hydrogen or a group selected from among phenyl, methylthiophenyl and nitrophenyl, and
R⁴ and R^{4'} which may be identical or different denote hydrogen or a group selected from among methyl, hydroxymethyl and phenyl,
with the proviso that if R³ and R^{3'} have the same meaning, the groups R⁴ and R^{4'} cannot have the same meaning, and with the proviso that if R⁴ and R^{4'} have the same meaning, the groups R³ and R^{3'} cannot have the same meaning,
to form a salt of formula **1-salt**, wherein the groups R¹, R², R³, R^{3'}, R⁴ and R^{4'} may have the meanings given hereinbefore, the resulting crystalline compound **1-salt** is isolated and the target compound **1** is liberated therefrom.

6. Compounds of formula **1-salt** 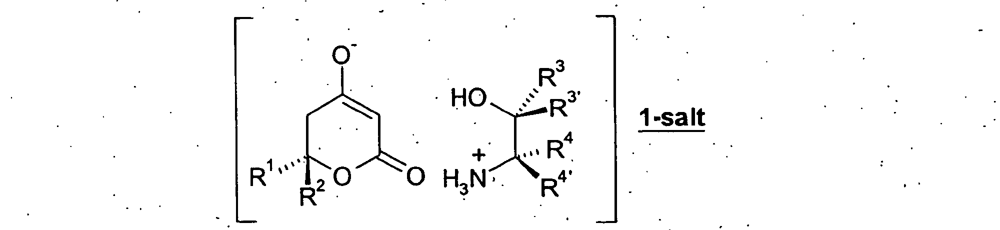 wherein
R¹ and R² independently of one another denote hydrogen or a group selected from among C₁-C₄-alkyl, C₃-C₈-cycloalkyl, C₆-C₁₀-aryl and -C₁-C₄-alkylene-C₆-C₁₀-aryl, which may optionally be mono-, di- or trisubstituted by one or more groups selected from among hydroxy, halogen, C₁-C₄-alkoxy and CF₃;
R³, R^{3'}, R⁴ and R^{4'} which may be identical or different denote hydrogen or a group selected from among C₁-C₄-alkyl, C₃-C₈-cycloalkyl, C₆-C₁₀-aryl and -C₁-C₄-alkylene-C₆-C₁₀-aryl, which may optionally be mono-, di- or trisubstituted by one or more groups selected from among hydroxy, halogen, C₁-C₄-alkoxy, -S-C₁-C₄-alkyl, -SO₂-C₁-C₄-alkyl, NO₂ and CF₃,
optionally in the form of the tautomers thereof,
with the proviso that R¹ and R² cannot simultaneously have the same meaning,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning.

7. Compounds of formula **1-salt** according to claim 6,
wherein
R¹ and R² independently of one another denote a group selected from among methyl, ethyl, propyl, butyl, phenyl, benzyl, phenylethyl and phenylpropyl which may optionally be monosubstituted by a group selected from among hydroxy, fluorine, chlorine, bromine, methoxy, ethoxy and CF₃;
R³, R^{3'}, R⁴ and R^{4'} which may be identical or different denote hydrogen or a group selected from among methyl, ethyl, propyl; phenyl; benzyl and phenylethyl, which may optionally be mono- or disubstituted by one or two groups selected from among hydroxy, fluorine, chlorine, bromine, methoxy, ethoxy, Me-S-, Me-SO₂-, Et-S-, Et-SO₂-, NO₂ and CF₃,
optionally in the form of the tautomers thereof,
with the proviso that R¹ and R² cannot simultaneously have the same meaning,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning.

8. Compounds of formula **1-salt** according to one of claims 6 or 7,
wherein
R¹ and R² independently of one another denote a group selected from among methyl, ethyl, propyl, butyl, phenyl, benzyl, phenylethyl and phenylpropyl,
R³, R^{3'}, R^{4'} and R^{4'} which may be identical or different denote hydrogen or a group selected from among methyl, ethyl, propyl, phenyl and benzyl, which may optionally be monosubstituted by a group selected from among hydroxy, methoxy, Me-S-, Me-SO₂- and NO₂,
optionally in the form of the tautomers thereof,
with the proviso that R¹ and R² cannot simultaneously have the same meaning,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning.

9. Compounds of formula **1-salt** according to one of claims 6, 7 or 8,
wherein
R¹ and R² independently of one another denote a group selected from among ethyl, propyl, butyl, benzyl, phenylethyl and phenylpropyl,
R³ and R^{3'} which may be identical or different denote hydrogen or a group selected from among phenyl, hydroxyphenyl, methylthiophenyl and nitrophenyl;
R⁴ and R^{4'} which may be identical or different denote hydrogen or a group selected from among methyl, hydroxymethyl and phenyl,
optionally in the form of the tautomers thereof,
with the proviso that R¹ and R² cannot simultaneously have the same meaning,
with the proviso that if R³ and R^{3'} have the same meaning, the groups R⁴ and R^{4'} cannot have the same meaning, and
with the proviso that if R⁴ and R^{4'} have the same meaning, the groups R³ and R^{3'} cannot have the same meaning.

10. Compound of formula **1-salt** according to one of claims 6 to 9,
wherein
R¹ denotes phenylethyl and R² denotes propyl,
R³ and R^{3'} which may be identical or different denote hydrogen or a group selected from among phenyl, methylthiophenyl and nitrophenyl, and
R⁴ and R^{4'} which may be identical or different denote hydrogen or a group selected from among methyl, hydroxymethyl and phenyl, optionally in the form of the tautomers thereof, with the proviso that if R³ and R^{3'} have the same meaning, the groups R⁴ and R^{4'} cannot have the same meaning, and with the proviso that if R⁴ and R^{4'} have the same meaning, the groups R³ and R^{3'} cannot have the same meaning.

11. Use of compounds of general formula **1-salt** according to one of claims 6 to 10 for the preparation of pharmaceutically active compounds.

12. Use of compounds of formula **1-salt** according to claim 11 for the preparation of tipranavir.

## Revendications

1. Procédé de production d'un composé de formule **1** 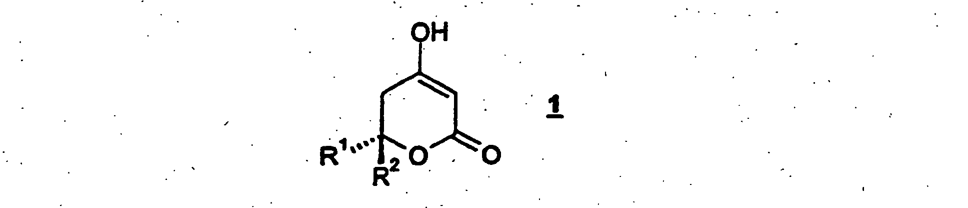 éventuellement sous forme de ses tautomères,
où
R¹ et R² représentent indépendamment l'un de l'autre l'hydrogène ou un groupement choisi dans le groupe consistant en C₁-C₄-alkyle, C₃-C₈-cycloalkyle, C₆-C₁₀-aryle et -C₁-C₄-alkylène-C₆-C₁₀-aryle, qui peut éventuellement être substitué une, deux ou trois fois par un ou plusieurs groupements choisis dans le groupe consistant en hydroxy, halogène, C₁-C₄-alcoxy et CF₃,
avec la condition que R¹ et R² ne peuvent pas présenter simultanément la même signification,
**caractérisé en ce que**, dans une première étape, un composé de formule **1-rac** 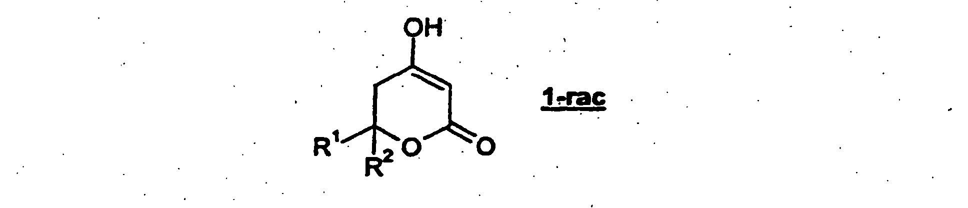 où R¹ et R² peuvent présenter les significations citées précédemment, est converti dans un solvant approprié avec un ou plusieurs, de préférence un aminoalcool chiral de formule **4** 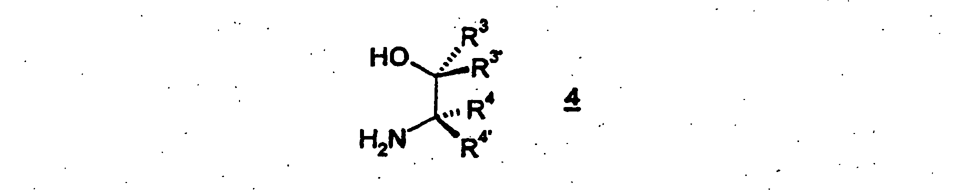 où
R³, R^{3'}, R⁴ et R^{4'}, identiques ou différents, représentent l'hydrogène ou un groupement choisi dans le groupe consistant en C₁-C₄-alkyle, C₃-C₈-cycloalkyle, C₆-C₁₀-aryle et -C₁-C₄-alkylène-C₆-C₁₀-aryle, qui peut éventuellement être substitué une, deux ou trois fois par un ou plusieurs groupements choisis dans le groupe consistant en hydroxy, halogène, C₁-C₄-alcoxy, -S-C₁-C₄-alkyle, -SO₂-C₁-C₄-alkyle, NO₂ et CF₃,
avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification,
en un sel de formule **1-salt** 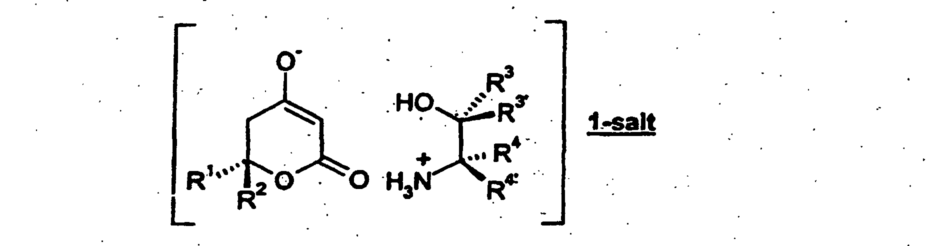 où les groupements R¹, R², R³, R^{3'}, R⁴ et R^{4'} peuvent avoir les significations citées précédemment, le composé cristallin **1-salt** obtenu est isolé et le composé cible est libéré à partir de celui-ci.

2. Procédé de production d'un composé de formule **1****,** éventuellement sous forme de ses tautomères, selon la revendication 1,
où
R¹ et R² représentent indépendamment l'un de l'autre un groupement choisi dans le groupe consistant en méthyle, éthyle, propyle, butyle, phényle, benzyle, phényléthyle et phénylpropyle qui peut éventuellement être substitué une fois par un groupement choisi dans le groupe consistant en hydroxy, fluor, chlore, brome, méthoxy, éthoxy et CF₃,
avec la condition que R¹ et R² ne peuvent pas présenter simultanément la même signification,
**caractérisé en ce que**, dans une première étape, un composé de formule **1-rac** où R¹ et R² peuvent présenter les significations citées précédemment, est converti dans un solvant approprié avec un ou plusieurs; de préférence un aminoalcool chiral de formule **4**
où
R³, R^{3'}, R⁴ et R^{4'}, identiques ou différents, représentent l'hydrogène ou un groupement choisi dans le groupe consistant en méthyle, éthyle, propyle, phényle, benzyle et phényléthyle, qui peut éventuellement être substitué une ou deux fois par un ou deux groupements choisis dans le groupe consistant en hydroxy, fluor, chlore, brome, méthoxy, éthoxy, Me-S-, Me-SO₂-, Et-S-, Et-SO₂-, NO₂ et CF₃, avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification,
en un sel de formule **1-salt** où les groupements R¹, R², R³, R^{3'}, R⁴ et R^{4'} peuvent avoir les significations citées précédemment, le composé cristallin **1-salt** obtenu est isolé et le composé cible **1** est libéré à partir de celui-ci.

3. Procédé de production d'un composé de formule **1**, éventuellement sous forme de ses tautomères, selon l'une des revendications 1 ou 2,
où
R¹ et R² représentent indépendamment l'un de l'autre un groupement choisi dans le groupe consistant en méthyle, éthyle, propyle, butyle, phényle, benzyle, phényléthyle et phénylpropyle,
avec la condition que R¹ et R² ne peuvent pas présenter simultanément la même signification,
**caractérisé en ce que**, dans une première étape, un composé de formule **1-rac** où R¹ et R² peuvent présenter les significations citées précédemment, est converti dans un solvant approprié avec un ou plusieurs, de préférence un aminoalcool chiral de formule **4**
où
R³, R^{3'}, R⁴ et R^{4'}, identiques ou différents, représentent l'hydrogène ou un groupement choisi dans le groupe consistant en méthyle, éthyle, propyle, phényle et benzyle, qui peut éventuellement être substitué une fois par un groupement choisi dans le groupe consistant en hydroxy, méthoxy, Me-S-, Me-SO₂- et NO₂,
avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification,
en un sel de formule **1-salt** où les groupements R¹, R², R³, R^{3'}, R⁴ et R^{4'} peuvent avoir les significations citées précédemment, le composé cristallin **1-salt** obtenu est isolé et le composé cible **1** est libéré à partir de celui-ci.

4. Procédé de production d'un composé de formule **1**, éventuellement sous forme de ses tautomères, selon l'une des revendications 1, 2 ou 3,
où
R¹ et R² représentent indépendamment l'un de l'autre un groupement choisi dans le groupe consistant en éthyle, propyle, butyle, benzyle, phényléthyle et phénylpropyle,
avec la condition que R¹ et R² ne peuvent pas présenter simultanément la même signification,
**caractérisé en ce que**, dans une première étape, un composé de formule **1-rac** où R¹ et R² peuvent présenter les significations citées précédemment, est converti dans un solvant approprié avec un ou plusieurs, de préférence un aminoalcool chiral de formule **4**, où
R³ et R^{3'}, identiques ou différents, représentent l'hydrogène ou un groupement choisi dans le groupe consistant en phényle, hydroxyphényle, méthylthiophényle et nitrophényle,
et
R⁴ et R^{4'}, identiques ou différents, représentent l'hydrogène ou un groupement choisi dans le groupe consistant en méthyle, hydroxyméthyle et phényle,
avec la condition que, quand R³ et R^{3'} présentent la même signification, les groupements R⁴ ef R^{4'} ne peuvent pas présenter la même signification, ainsi qu'avec la condition que, quand R⁴ et R^{4'} présentent la même signification, les groupements R³ et R^{3'} ne peuvent pas présenter la même signification,
en un sel de formule **1-salt** où les groupements R¹, R², R³, R^{3'}, R⁴ et R^{4'} peuvent avoir les significations citées précédemment, le composé cristallin **1-salt** obtenu est isolé et le composé cible **1** est libéré à partir de celui-ci.

5. Procédé de production d'un composé de formule **1**, éventuellement sous forme de ses tautomères, selon l'une des revendications 1, 2, 3 ou 4,
où
R¹ représente phényléthyle et R² représente propyle,
**caractérisé en ce que**, dans une première étape, un composé de formule **1-rac** où R¹ et R² peuvent présenter les significations citées précédemment, est converti dans un solvant approprié avec un ou plusieurs, de préférence un aminoalcool chiral de formule **4**
où
R³ et R^{3'}, identiques ou différents, représentent l'hydrogène, ou un groupement choisi dans le groupe consistant en phényle, méthylthiophényle et nitrophényle,
et
R⁴ et R^{4'}, identiques ou différents, représentent l'hydrogène ou un groupement choisi dans le groupe consistant en méthyle, hydroxyméthyle et phényle,
avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification,
en un sel de formule **1-salt** où les groupements R¹, R², R³, R^{3'}, R⁴ et R^{4'} peuvent avoir les significations citées précédemment, le composé cristallin **1-salt** obtenu est isolé et le composé cible **1** est libéré à partir de celui-ci.

6. Composés de formule **1-salt** 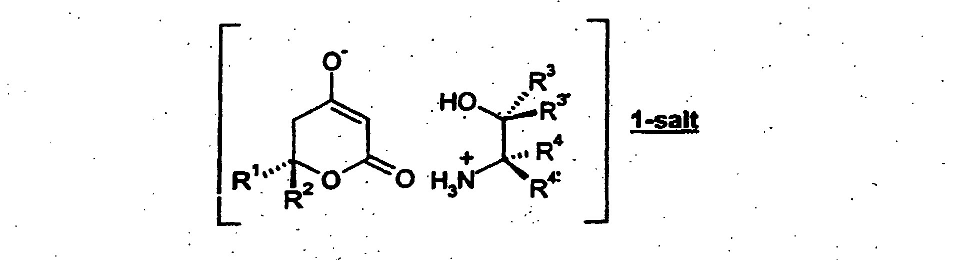 où
R¹ et R² représentent indépendamment l'un de l'autre l'hydrogène ou un groupement choisi dans le groupe consistant en C₁-C₄-alkyle, C₃-C₈-cycloalkyle, C₆-C₁₀-aryle et -C₁-C₄-alkylène-C₆-C₁₀-aryle, qui peut éventuellement être substitué une, deux ou trois fois par un ou plusieurs groupements choisis dans le groupe consistant en hydroxy, halogène, C₁-C₄-alcoxy et CF₃ ;
R³, R^{3'}, R⁴ et R^{4'}, identiques ou différents, représentent l'hydrogène ou un groupement choisi dans le groupe consistant en C₁-C₄-alkyle, C₃-C₈-cycloalkyle, C₆-C₁₀-aryle et -C₁-C₄-alkylène-C₆-C₁₀-aryle, qui peut éventuellement être substitué une, deux ou trois fois par un ou plusieurs groupements choisis dans le groupe consistant en hydroxy, halogène, C₁-C₄-alcoxy, -S-C₁-C₄-alkyle, -SO₂-C₁-C₄-alkyle, NO₂ et CF₃,
éventuellement sous forme de leurs tautomères,
avec la condition que R¹ et R² ne peuvent pas présenter simultanément la même signification, avec la condition que, quand R³, et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification.

7. Composés de formule **1-salt** selon la revendication 6
où
R¹ et R² représentent indépendamment l'un de l'autre un groupement choisi dans le groupe consistant en méthyle, éthyle, propyle, butyle, phényle, benzyle, phényléthyle et phénylpropyle qui peut éventuellement être substitué une fois par un groupement choisi dans le groupe consistant en hydroxy, fluor, chlore, brome, méthoxy, éthoxy et CF₃ ;
R³, R^{3'}, R⁴ et R^{4'} ; identiques ou différents, représentent l'hydrogène ou un groupement choisi dans le groupe consistant en méthyle, éthyle, propyle, phényle, benzyle et phényléthyle, qui peut éventuellement être substitué une ou deux fois par un ou deux groupements choisis dans le groupe consistant en hydroxy, fluor, chlore, brome, méthoxy, éthoxy, Me-S-, Me-SO₂-, Et-S-, Et-SO₂-, NO₂ et CF₃,
éventuellement sous forme de leurs tautomères,
avec la condition que R¹ et R² ne peuvent pas présenter simultanément la même signification, avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification.

8. Composés de formule **1-salt** selon l'une des revendications 6 ou 7
où
R¹ et R² représentent indépendamment l'un de l'autre un groupement choisi dans le groupe consistant en méthyle, éthyle, propyle, butyle, phényle, benzyle, phényléthyle et phénylpropyle,
R³, R^{3'}, R⁴ et R^{4'}, identiques ou différents, représentent l'hydrogène ou un groupement choisi dans le groupe consistant en méthyle, éthyle, propyle, phényle et benzyle, qui peut éventuellement être substitué une fois par un groupement choisi dans le groupe consistant en hydroxy, méthoxy, Me-S-, Me-SO₂- et NO₂,
éventuellement sous forme de leurs tautomères,
avec la condition que R¹ et R² ne peuvent pas présenter simultanément la même signification, avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification.

9. Composés de formule **1-salt** selon l'une des revendications 6, 7 ou 8
où
R¹ et R² représentent indépendamment l'un de l'autre un groupement choisi dans le groupe consistant en éthyle, propyle, butyle, benzyle, phényléthyle et phénylpropyle,
R³ et R^{3'}, identiques ou différents, représentent l'hydrogène ou un groupement choisi dans le groupe consistant en phényle, hydroxyphényle, méthylthiophényle et nitrophényle ;
R⁴ et R^{4'}, identiques ou différents, représentent l'hydrogène ou un groupement choisi dans le groupe consistant en méthyle, hydroxyméthyle et phényle,
éventuellement sous forme de leurs tautomères,
avec la condition que R¹ et R² ne peuvent pas présenter simultanément la même signification, avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification.

10. Composés de formule **1-salt** selon l'une des revendications 6 à 9
où
R¹ représente phényléthyle et R² représente propyle,
R³ et R^{3'}, identiques ou différents, représentent l'hydrogène ou un groupement choisi dans le groupe consistant en phényle, méthylthiophényle et nitrophényle ;
et
R⁴ et R^{4'}, identiques ou différents, représentent l'hydrogène ou un groupement choisi dans le groupe consistant en méthyle, hydroxyméthyle et phényle, éventuellement sous forme de leurs tautomères, avec la condition que, quand R³ et R^{3'} présentent la même signification, les groupements R⁴ et R^{4'} ne peuvent pas présenter la même signification, ainsi qu'avec la condition que, quand R⁴ et R^{4'} présentent la même signification, les groupements R³ et R^{3'} ne peuvent pas présenter la même signification.

11. Utilisation de composés de formule générale **1-salt** selon l'une des revendications 6 à 10 pour la production de composés pharmaceutiquement efficaces.

12. Utilisation de composés de formule **1-salt** selon la revendication 11 pour la production du tipranavir.
